# EUROPEAN PATENT APPLICATION

(11) **EP 4 364 647 A1**
(43) Date of publication of application: **08.05.2024**
(21) Application number: 22832915.7
(22) Date of filing: 20.06.2022
(51) Int. Cl.: A61B 5/00, A61B 5/107, G06Q 50/10

(54) **METHOD FOR DETERMINING REASON FOR CHANGE IN UNDER-EYE SAGGING, DEVICE FOR DETERMINING REASON FOR CHANGE IN UNDER-EYE SAGGING, PROGRAM, METHOD FOR EVALUATING UNDER-EYE SAGGING, DEVICE FOR EVALUATING UNDER-EYE SAGGING, AND PROGRAM**

(30) Priority: 28.06.2021 JP 2021107017; 28.06.2021 JP 2021107018
(71) Applicant: Shiseido Company, Ltd., Chuo-ku Tokyo 104-0061 (JP)
(72) Inventor: TAKAI, Eisuke, Tokyo 104-0061 (JP)
(74) Representative: Ter Meer Steinmeister & Partner
(86) International application number: PCT/JP2022/024607
(87) International publication number: WO 2023/276767

(57) **Abstract**

The reason why under-eye sagging has changed is determined. A method according to an embodiment of the present invention includes acquiring information on a three-dimensional shape of an under-eye area of a subject when the subject is in a vertical position before a change in under-eye sagging of the subject and information on a three-dimensional shape of the under-eye area of the subject when the subject is in the vertical position after the change in the under-eye sagging; and determining a reason for the change in the under-eye sagging, based on a volume difference between the three-dimensional shape of the under-eye area of the subject when the subject is in the vertical position before the change in the under-eye sagging and the three-dimensional shape of the under-eye area of the subject when the subject is in the vertical position after the change in the under-eye sagging.

## Description

### TECHNICAL FIELD

The present invention relates to a reason-for-change in under-eye sagging determination method, a reason-for-change in under-eye sagging determination apparatus, and a program, an under-eye sagging evaluation method, an under-eye sagging evaluation apparatus, and a program.

### BACKGROUND ART

Conventionally, methods of evaluating effects of cosmetics, beauty treatments, and the like are known. For example, Patent Document 1 discloses a method in which coordinates representing the degree of swelling around the eyes after a massage or aesthetic treatment are subtracted from coordinates representing the degree of swelling around the eyes before the treatment. In this method, if a subtraction result is positive, a corresponding portion is displayed brightly, and if the subtraction result is zero or negative, a corresponding portion is displayed darkly in accordance with its degree.

Further, methods of evaluating under-eye sagging are known. For example, Non-Patent Document 1 reports a method of visually evaluating under-eye sagging on a seven-grade score from 0 to 6.

### RELATED-ART DOCUMENTS

### PATENT DOCUMENTS

Patent Document 1: Japanese Patent No. 3853720

### NON-PATENT DOCUMENTS

Non-Patent Document 1: T. Ezure et. al., Skin Res. Tech., 17, 510-515(2011)

### SUMMARY OF THE INVENTION

### PROBLEM TO BE SOLVED BY THE INVENTION

However, in Patent Document 1, only subtraction results of coordinates representing the degrees of swelling around the eyes are displayed, and the reason why under-eye sagging has changed cannot be determined. Therefore, it has been desired to determine the reason why under-eye sagging has changed.

Further, the reason why the face appears to sag has not been sufficiently understood. Therefore, an evaluation based on appropriate factors that cause a sagging appearance has been desired.

In view of the above, it is an object of the present invention to determine the reason why under-eye sagging has changed.

In addition, it is an object of the present invention to find factors that cause an under-eye sagging appearance, and evaluate sagging under-eye sagging based on the found factors.

### MEANS TO SOLVE THE PROBLEM

A method according to an aspect of the present invention includes acquiring information on a three-dimensional shape of an under-eye area of a subject when the subject is in a vertical position before a change in under-eye sagging of the subject and information on a three-dimensional shape of the under-eye area of the subject when the subject is in the vertical position after the change in the under-eye sagging; and determining a reason for the change in the under-eye sagging, based on a volume difference between the three-dimensional shape of the under-eye area of the subject when the subject is in the vertical position before the change in the under-eye sagging and the three-dimensional shape of the under-eye area of the subject when the subject is in the vertical position after the change in the under-eye sagging.

A method according to an aspect of the present invention includes acquiring information on a three-dimensional shape of an under-eye area including an upper cheek of a subject when the subject is in a horizontal position and information on a three-dimensional shape of the under-eye area including the upper cheek of the subject when the subject is in a vertical position; evaluating at least one of under-eye swelling and drooping of the upper cheek based on a difference between the three-dimensional shape of the under-eye area including the upper cheek of the subject when the subject is in the horizontal position and the three-dimensional shape of the under-eye area including the upper cheek of the subject when the subject is in the vertical position; and performing an evaluation of under-eye sagging based on at least one of the under-eye swelling and the drooping of the upper cheek.

### EFFECTS OF THE INVENTION

According to the present invention, the reason why under-eye sagging has changed can be determined.

In addition, factors that cause an under-eye sagging appearance can be found, and under-eye sagging can be evaluated based on the found factors.

### BRIEF DESCRIPTION OF THE DRAWINGS

[FIG. 1] FIG. 1 is a drawing illustrating a shape-up improvement type and a pump-up improvement type according to an embodiment of the present invention;
[FIG. 2] FIG. 2 is a drawing illustrating a shape-up improvement type and a pump-up improvement type as a result of daily skin care using basic cosmetics according to an embodiment of the present invention;
[FIG. 3] FIG. 3 is a drawing illustrating the shape-up improvement type and the pump-up improvement type as a result of a daily scalp massage according to an embodiment of the present invention;
[FIG. 4] FIG. 4 is a drawing illustrating the shape-up improvement type and the pump-up improvement type as a result daily skin care using a radio frequency (RF)/electronic muscle stimulation (EMS) facial care device according to an embodiment of the present invention;
[FIG. 5] FIG. 5 is a block diagram of a reason-for-change in under-eye sagging determination system according to an embodiment of the present invention;
[FIG. 6] FIG. 6 is a functional block diagram of a reason-for-change in under-eye sagging determination apparatus 10 according to an embodiment of the present invention;
[FIG. 7] FIG. 7 is a sequence diagram of a process of determining the reason for a change in under-eye sagging according to an embodiment of the present invention;
[FIG. 8] FIG. 8 is a flowchart of the process of determining the reason for the change in the under-eye sagging according to an embodiment of the present invention;
[FIG. 9] FIG. 9 is an example in which the correlation between the reasons for changes in under-eye sagging, determined for a plurality of subjects, and answers to a predetermined questionnaire by the plurality of subjects is generated, and the correlation is used to estimate the reason for a change in under-eye sagging based on answers to the predetermined questionnaire according to an embodiment of the present invention;
[FIG. 10] FIG. 10 is a drawing illustrating under-eye swelling;
[FIG. 11] FIG. 11 is a drawing illustrating under-eye swelling;
[FIG. 12] FIG. 12 is a drawing illustrating the relationship between an under-eye sagging score and age;
[FIG. 13] FIG. 13 is a drawing illustrating drooping of the upper cheek;
[FIG. 14] FIG. 14 is a drawing illustrating the relationship between an under-eye sagging score and drooping of the upper cheek;
[FIG. 15] FIG. 15 is a drawing illustrating, together with regression equations, the relationship between age and the amount of drooping of the upper cheek and the relationship between the amount of drooping of the upper cheek and an under-eye sagging score;
[FIG. 16] FIG. 16 is a drawing illustrating the relationship between an under-eye sagging score versus under-eye swelling and drooping of the upper cheek;
[FIG. 17] FIG. 17 is a drawing illustrating estimation of the amount of drooping of the upper cheek based on one or both of age and an under-eye sagging score;
[FIG. 18] FIG. 18 is a drawing illustrating under-eye sagging and under-eye dark circles; [FIG. 19] FIG. 19 is a block diagram of an under-eye sagging evaluation system according to an embodiment of the present invention;
[FIG. 20] FIG. 20 is a functional block diagram of an under-eye sagging evaluation apparatus according to an embodiment of the present invention;
[FIG. 21] FIG. 21 is a sequence diagram of a process of evaluating under-eye sagging according to an embodiment of the present invention;
[FIG. 22] FIG. 22 is a flowchart of the process of evaluating under-eye sagging according to an embodiment of the present invention; and
[FIG. 23] FIG. 23 is a block diagram illustrating an example of a hardware configuration of the reason-for-change in under-eye sagging determination apparatus.

### MODE FOR CARRYING OUT THE INVENTION

In the following, embodiments of the present invention will be described with reference to the accompanying drawings.

### <Description of Terms>

- In the present specification, the term "under-eye area" refers to an area of a face defined by a lower eyelid serving as the upper limit, a cheekbone serving as the lower limit, and a region between a nose and a sideburn in the lateral direction. There are two under-eye areas on both sides of the nose.
- In the present specification, the term "horizontal position" refers to a state in which the median plane of the face rests at right angles to the direction of gravity.
- In the present specification, the term "vertical position" refers a state in which the median plane of the face rests parallel to the direction of gravity.
- In the present specification, the term "change in under-eye sagging" refers to a change in the state of the under-eye area. For example, a change in under-eye sagging is an improvement of under-eye sagging.

### [Types of Changes in Under-Eye Sagging]

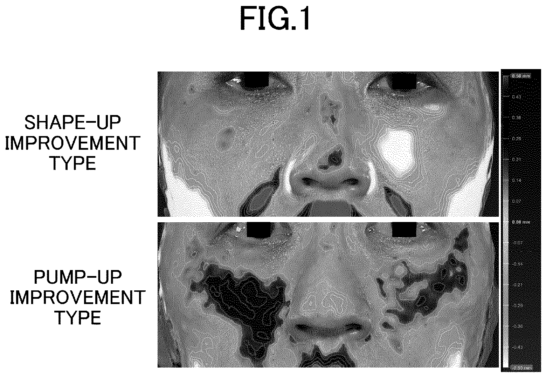

Types of changes of under-eye sagging will be described with reference to FIG. 1 through FIG. 4.

FIG. 1 is a drawing illustrating a shape-up improvement type and a pump-up improvement type according to an embodiment of the present invention.

For a plurality of subjects who experienced changes in under-eye sagging (specifically, improvements in under-eye sagging), differences (that is, changes) between the three-dimensional shapes of the under-eye areas of the subjects when the subjects were in the vertical position before the changes in the under-eye sagging and the three-dimensional shapes of the under-eye areas of the subjects when the subjects were in the vertical position after the changes in the under-eye sagging were examined. As a result, the subjects were classified into three groups: those whose volumes of the under-eye areas were smaller after the changes in the under-eye sagging than before the changes in the under-eye sagging (hereinafter referred to as a "shape-up improvement type" as illustrated in the upper part of FIG. 1); those whose volumes of the under-eye areas were larger after the changes in the under-eye sagging than before the changes in the under-eye sagging (hereinafter also referred to as a "pump-up improvement type" as illustrated in the lower part of FIG. 1); and those whose volumes of the under-eye areas were the same before the changes in the under-eye sagging and after the changes in the under-eye sagging (hereinafter referred to as a dark-circle improvement type).

In the shape-up improvement type, it is considered that the under-eye sagging has changed (for example, improved) as a result of the disappearance of edema. Further, in the pump-up improvement type, it is considered that the under-eye sagging has changed (for example, improved) as a result of improved elasticity. Further, in the dark-circle improvement type, it is considered that the under-eye sagging has changed (for example, improved) as a result of changes in under-eye skin colors including at least one of under-eye dark circles and pigmentation (hereinafter also simply referred to as under-eye dark circles and pigmentation).

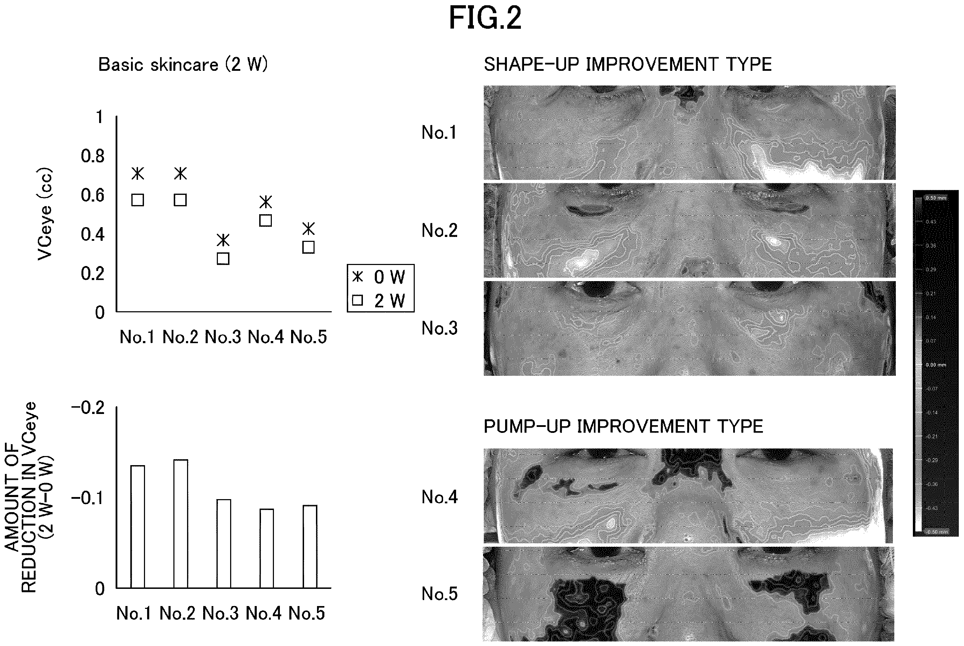

FIG. 2 is a drawing illustrating the shape-up improvement type and the pump-up improvement type as a result of using basic cosmetics according to an embodiment of the present invention. For each of subjects (No. 1 to No. 5) who experienced changes in under-eye sagging as a result of using the basic cosmetics, "drooping (VCeye) of the upper cheek" was examined before the change and after the change (two weeks after the change). Note that "drooping of the upper cheek" means that the upper cheek is more hollow when each of the subjects is in the vertical position than when in the horizontal position.

As indicated in the upper left graph of FIG. 2, drooping of the upper cheek of each of the subjects (No. 1 to No. 5) was more reduced after the change than before the change. In addition, the amount of reduction in drooping of the upper cheek ("the amount (VCeye) of drooping of the upper cheek after the change" - "the amount (VCeye) of drooping of the upper cheek before the change") of each of the subjects was as indicated in the lower left graph of FIG. 2. The subjects were classified into groups: those (subjects No. 1 to No. 3) of the shape-up improvement type; and those (subjects No. 4 and No. 5) of the pump-up improvement type.

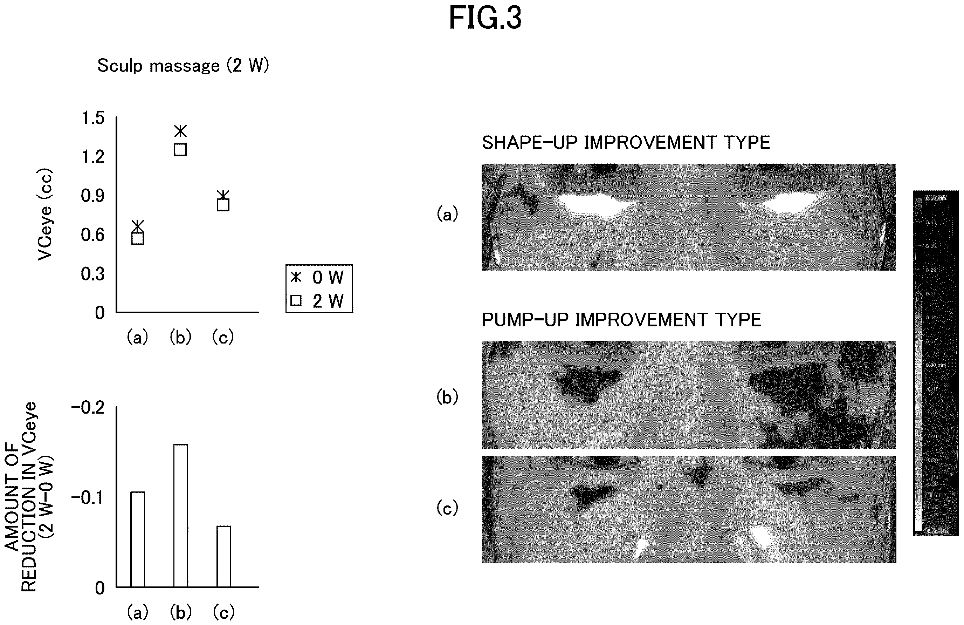

FIG. 3 is a drawing illustrating the shape-up improvement type and the pump-up improvement type as a result of a scalp massage according to an embodiment of the present invention. For each of subjects ((a) to (c)) who experienced changes in under-eye sagging as a result of the scalp massage, "drooping (VCeye) of the upper cheek" was examined before the change and after the change (two weeks after the change). Note that "drooping of the upper cheek" means that the upper cheek is more hollow when each of the subjects is in the vertical position than when the subject is in the horizontal position.

As indicated in the upper left graph of FIG. 3, drooping of the upper cheek of each of the subjects ((a) to (c)) was more reduced after the change than before the change. In addition, the amount of reduction in drooping of the upper cheek ("the amount (VCeye) of drooping of the upper cheek after the change" - "the amount (VCeye) of drooping of the upper cheek before the change") of each of the subjects was as indicated in the lower left graph of FIG. 3. The subjects were classified into two groups: those (subject (a)) of the shape-up improvement type; and those (subjects (b) and (C)) of the pump-up improvement type.

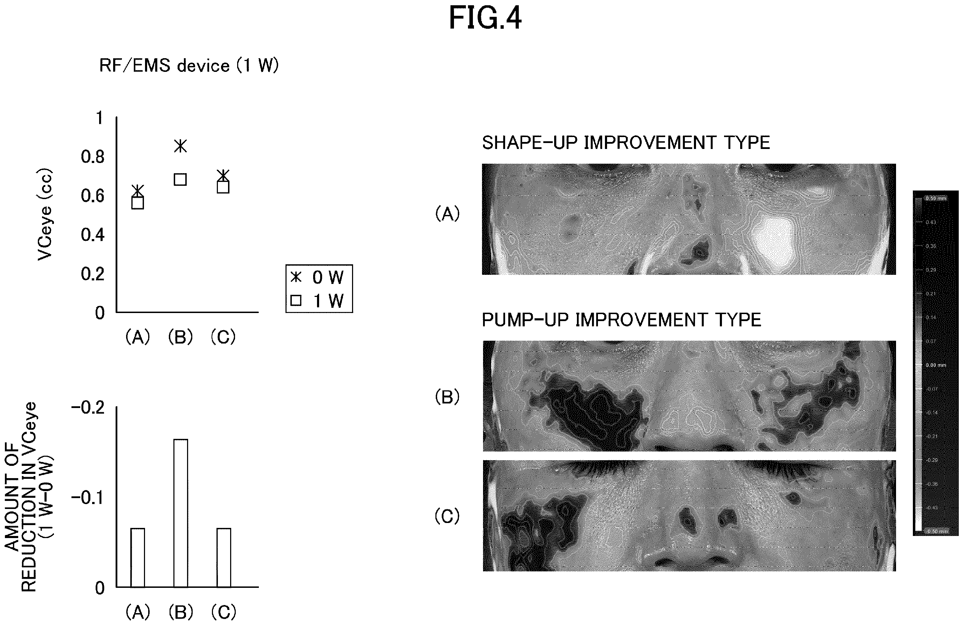

FIG. 4 is a drawing illustrating the shape-up improvement type and the pump-up improvement type as a result of using a radio frequency (RF)/electronic muscle stimulation (EMS) facial care device according to an embodiment of the present invention. For each of subjects ((A) to (C)) who experienced changes in under-eye sagging as a result of using the RF-EMS facial care device, "drooping (VCeye) of the upper cheek" was examined before the change and after the change (one week after the change). Note that "drooping of the upper cheek" means that the upper cheek is more hollow when each of the subjects is in the vertical position than when the subject is in the horizontal position.

As indicated in the upper left graph of FIG. 4, drooping of the upper cheek of each of the subjects ((A) to (C)) was more reduced after the change than before the change. In addition, the amount of reduction in drooping of the upper cheek ("the amount (VCeye) of drooping of the upper cheek after the change" - "the amount (VCeye) of drooping of the upper cheek before the change") of each of the subjects was as indicated in the lower left graph of FIG. 4. The subjects were classified into two groups: those (subject A) of the shape-up improvement type, and those (subjects (b) and (C)) of the pump-up improvement type.

As described above, from the examination results illustrated in FIG. 1 to FIG. 4, it has been found that the reason for a change in under-eye sagging can be determined based on a volume difference between the three-dimensional shape of the under-eye area of a subject when the subject is in the vertical position before the change in the under-eye sagging and the three-dimensional shape of the under-eye area of the subject when the subject is in the vertical position after the change in the under-eye sagging.

### [Determination of Reason (Factor) for Change in Under-eye sagging]

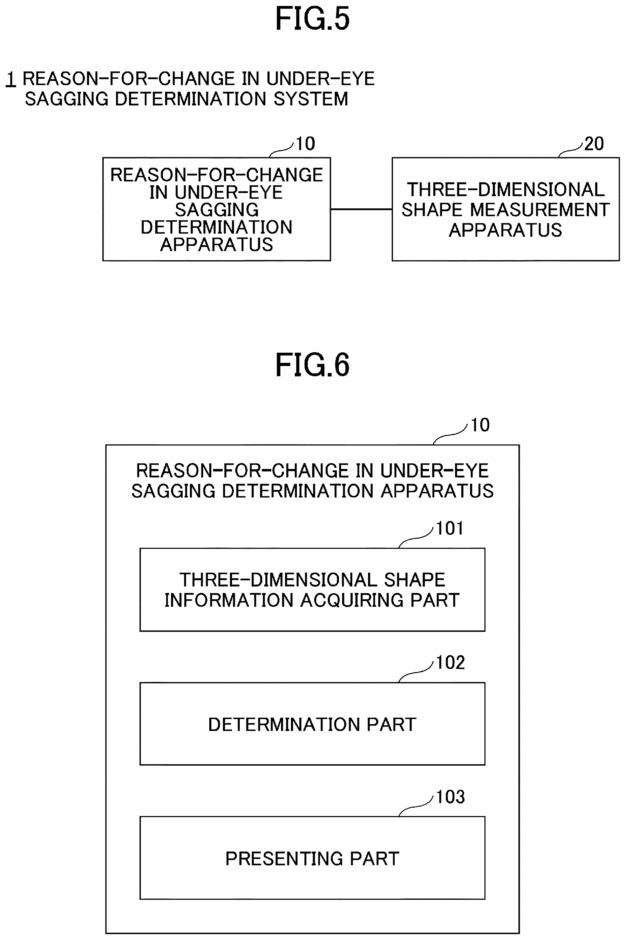

Determination of the reason for a change in under-eye sagging of a subject (a person for whom the reason for a change in under-eye sagging is to be determined) will be described with reference to FIG. 5 to FIG. 8. Specifically, a description is given of determination of the reason for a change in under-eye sagging based on whether the volume of the under-eye area is smaller after the change in the under-eye sagging than before the change in the under-eye sagging, whether the volume of the under-eye area is larger after the change in the under-eye sagging than before the change in the under-eye sagging, or whether the volume of the under-eye area is the same before the change in the under-eye sagging and after the change in the under-eye sagging.

### <Overall Configuration>

FIG. 5 is a block diagram of a reason-for-change in under-eye sagging determination system 1 according to an embodiment of the present invention. The reason-for-change in under-eye sagging determination system 1 includes a reason-for-change in under-eye sagging determination apparatus 10 and a three-dimensional shape measurement apparatus 20.

The reason-for-change in under-eye sagging determination apparatus 10 is a computer (for example, a personal computer, a tablet, a smartphone, a server, or the like) configured to determine the reason for a change in under-eye sagging. Specifically, the reason-for-change in under-eye sagging determination apparatus 10 is configured to determine the reason for a change in under-eye sagging based on a difference (that is, a change) between the three-dimensional shape of the under-eye area of a subject when the subject is in the vertical position before the change in the under-eye sagging and the three-dimensional shape of the under-eye area of the subject when the subject is in the vertical position after the change in the under-eye sagging. For example, the day on which the three-dimensional shape of the under-eye area of the subject is measured before the change in the under-eye sagging and the day on which the three-dimensional shape of the under-eye area of the subject is measured after the change in the under-eye sagging are different days. The reason-for-change in under-eye sagging determination apparatus 10 will be described later in detail with reference to FIG. 6.

Although the reason-for-change in under-eye sagging determination apparatus 10 is described as one computer in FIG. 5, the reason-for-change in under-eye sagging determination apparatus 10 may be implemented by a plurality of computers (for example, a terminal such as a smartphone used by the subject, and a server that can transmit/receive data to/from the terminal. For example, the terminal such as the smartphone used by the subject may transmit, to the server, information on the three-dimensional shapes of the under-eye area of the subject when the subject is in the vertical position before and after the change in the under-eye sagging, and may receive, from the server, a determination result of the reason for the change in the under-eye sagging determined by the server.

The three-dimensional shape measurement apparatus 20 is an apparatus configured to measure the three-dimensional shape of the under-eye area of the subject. The three-dimensional shape measurement apparatus 20 can generate an image representing the three-dimensional shape of the face including at least the under-eye area of the subject. Specifically, the three-dimensional shape measurement apparatus 20 generates an image representing the three-dimensional shape of the under-eye area of the subject when the subject is in the vertical position before the change in the under-eye sagging of the subject, and an image representing the three-dimensional shape of the under-eye area of the subject when the subject is in the vertical position after the change in the under-eye sagging.

### <Functional Block>

FIG. 6 is a functional block diagram of the reason-for-change in under-eye sagging determination apparatus 10 according to an embodiment of the present invention. The reason-for-change in under-eye sagging determination apparatus 10 can include a three-dimensional shape information acquiring part 101, a determination part 102, and a presenting part 103. Further, the reason-for-change in under-eye sagging determination apparatus 10 can function as the three-dimensional shape information acquiring part 101, the determination part 102, and the presenting part 103 by executing a program. Each of the parts will be described below.

The three-dimensional shape information acquiring part 101 acquires information on the three-dimensional shape of the under-eye area of the subject when the subject is in the vertical position before the change in the under-eye sagging of the subject and information on the three-dimensional shape of the under-eye area of the subject when the subject is in the vertical position after the change in the under-eye sagging. For example, the three-dimensional shape information acquiring part 101 acquires, from the three-dimensional shape measurement apparatus 20, an image representing the three-dimensional shape of the under-eye area of the subject when the subject is in the vertical position before the change in the under-eye sagging of the subject, and an image representing the three-dimensional shape of the under-eye area of the subject when the subject is in the vertical position after the change in the under-eye sagging of the subject. The three-dimensional shape information acquiring part 101 stores the acquired information on the three-dimensional shapes of the under-eye area of the subject in a memory so that the determination part 102 can refer to the information.

The determination part 102 determines the reason for the change in the under-eye sagging of the subject based on the information on the three-dimensional shapes of the under-eye area of the subject acquired by the three-dimensional shape information acquiring part 111. Specifically, the determination part 102 determines the reason for the change in the under-eye sagging, based on a volume difference between the three-dimensional shape of the under-eye area of the subject when the subject is in the vertical position before the change in the under-eye sagging and the three-dimensional shape of the under-eye area of the subject when the subject is in the vertical position after the change in the under-eye sagging. Three reasons will be described below.

### <Shape-Up Improvement Type>

In a case where the volume of the under-eye area is smaller after the change in the under-eye sagging than before the change in the under-eye sagging, the determination part 102 determines that the under-eye sagging has changed as a result of the disappearance of edema originally observed in the subject and causing the under-eye sagging.

### <Pump-Up Improvement Type>

In a case where the volume of the under-eye area is larger after the change in the under-eye sagging of the subject than before the change in the under-eye sagging, the determination part 102 determines that the under-eye sagging has changed as a result of an improvement in loss of elasticity originally observed in the subject and causing the under-eye sagging.

### <Dark-Circle Improvement Type>

In a case where the volume of the under-eye area cannot be determined to be larger or smaller after the change in the under-eye sagging than before the change in the under-eye sagging, the determination part 102 determines that the under-eye sagging has changed as a result of a change in an under-eye skin color, including at least one of an under-eye dark circle and pigmentation, originally observed in the subject and causing the under-eye sagging.

The presenting part 103 can present a determination result by the determination part 102 (for example, display a determination result on a display unit of the reason-for-change in under-eye sagging determination apparatus 10 or another apparatus).

### <Method>

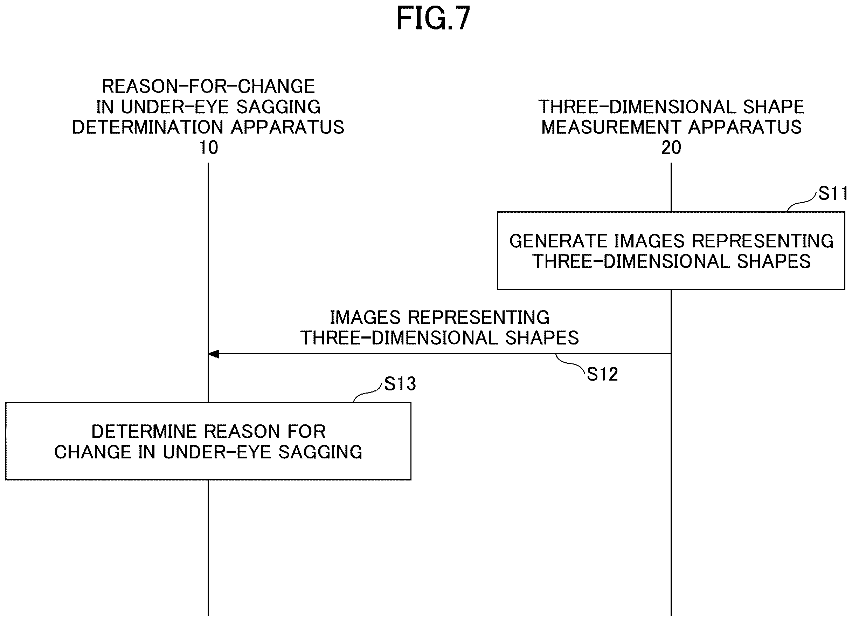

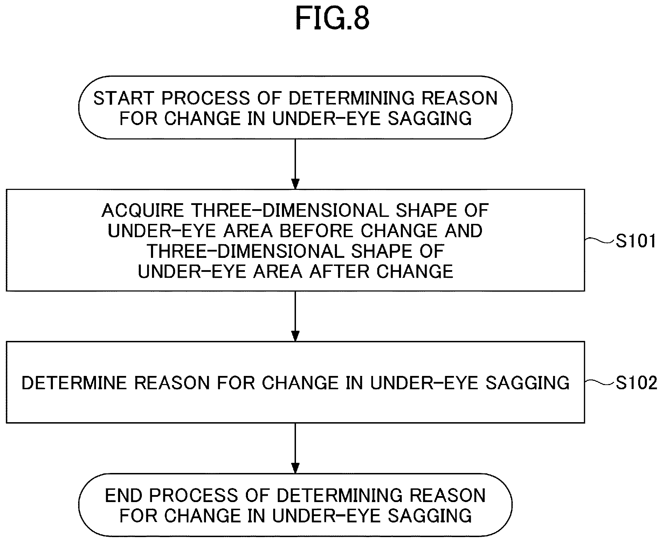

A method of determining the reason of a change in under-eye sagging will be described below with reference to FIG. 7 and FIG. 8.

FIG. 7 is a sequence diagram of a process of determining the reason for a change in under-eye sagging according to an embodiment of the present invention.

In step 11 (S 1 1), the three-dimensional shape measurement apparatus 20 generates an image representing the three-dimensional shape of the under-eye area of a subject when the subject is in the vertical position before a change in under-eye sagging of the subject, and an image representing the three-dimensional shape of the under-eye area of the subject when the subject is in the vertical position after the change in the under-eye sagging of the subject.

In step 12 (S12), the reason-for-change in under-eye sagging determination apparatus 10 acquires, from the three-dimensional shape measurement apparatus 20, the image (image generated in S11) representing the three-dimensional shape of the under-eye area of the subject when the subject is in the vertical position before the change in the under-eye sagging of the subject, and the image (image generated in S11) representing the three-dimensional shape of the under-eye area of the subject when the subject is in the vertical position after the change in the under-eye sagging.

In step 13 (S13), the reason-for-change in under-eye sagging determination apparatus 10 determines the reason for the change in the under-eye sagging of the subject based on information on the three-dimensional shapes of the under-eye area of the subject, which are acquired in S12.

FIG. 8 is a flowchart of the process of determining the reason for the change in the under-eye sagging according to an embodiment of the present invention.

In step 101 (S101), the three-dimensional shape information acquiring part 101 of the reason-for-change in under-eye sagging determination apparatus 10 acquires the information on the three-dimensional shape of the under-eye area of the subject when the subject is in the vertical position before the change in the under-eye sagging of the subject and the information on the three-dimensional shape of the under-eye area of the subject when the subject is in the vertical position after the change in the under-eye sagging. For example, the three-dimensional shape information acquiring part 101 acquires, from the three-dimensional shape measurement apparatus 20, the image representing the three-dimensional shape of the under-eye area of the subject when the subject is in the vertical position before the change in the under-eye sagging of the subject, and the image representing the three-dimensional shape of the under-eye area of the subject when the subject is in the vertical position after the change in the under-eye sagging.

In step 102 (S102), the determination part 102 of the reason-for-change in under-eye sagging determination apparatus 10 determines the reason for the change in the under-eye sagging, based on a volume difference between the three-dimensional shape of the under-eye area of the subject when the subject is in the vertical position before the change in the under-eye sagging and the three-dimensional shape of the under-eye area of the subject when the subject is in the vertical position after the change in the under-eye sagging, which are acquired in S101.

Specifically, in a case where the volume of the under-eye area is smaller after the change in the under-eye sagging than before the change in the under-eye sagging, the determination part 102 determines that the under-eye sagging has changed as a result of the disappearance of edema. Further, in a case where the volume of the under-eye area is larger after the change in the under-eye sagging than before the change in the under-eye sagging, the determination part 102 determines the under-eye sagging has changed as a result of an improvement in loss of elasticity. Further, in a case where the volume of the under-eye area is the same before the change in the under-eye sagging and after the change in the under-eye sagging, the determination part 102 determines that the under-eye sagging has changed as a result of the disappearance of a dark circle.

### <Other Embodiments>

According to an embodiment of the present invention, the correlation between the reasons for changes in under-eye sagging determined for a plurality of subjects and answers to a predetermined questionnaire (for example, a questionnaire about under-eye sagging) given by the plurality of subjects can be generated, and the correlation can be used to estimate the reason for a change in under-eye sagging of a person based on answers to the predetermined questionnaire. Specifically, the reason-for-change in under-eye sagging determination apparatus 10 can acquire answers to the predetermined questionnaire from a person for whom the reason for a change in under-eye sagging is to be estimated, and can use the correlation to estimate the reason for a change in under-eye sagging of the person based on the answers to the predetermined questionnaire.

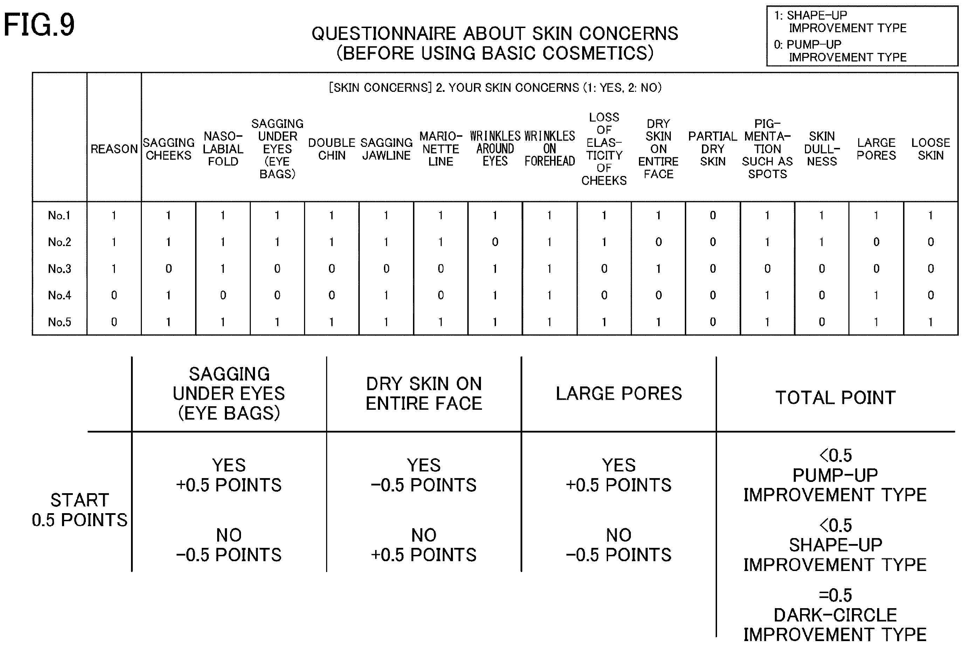

FIG. 9 is an example in which the correlation between the reasons for changes in under-eye sagging, determined for a plurality of subjects, and answers to a predetermined questionnaire by the plurality of subjects is generated, and the correlation is used to estimate the reason for a change in under-eye sagging based on answers to the predetermined questionnaire. In this example, for the subjects (No. 1 to No. 5) who have experienced changes in under-eye sagging as a result of using the basic cosmetics as described with reference to FIG. 2, a questionnaire about skin concerns was conducted before the changes in the under-eye sagging.

The lower part of FIG. 9 is an algorithm chart for estimating the reason for a change in under-eye sagging based on answers to the questionnaire about the skin concerns conducted before the change. In this algorithm chart, answers to the questionnaire about the skin concerns given by the subjects and the actually determined reasons for the changes in the under-eye sagging are used as training data. By using the algorithm, for a person other than the subjects (No. 1 to No. 5) who have experienced the changes in the under-eye sagging as a result of using the basic cosmetics, a reason for a change in under-eye sagging as a result of daily skin care using the basic cosmetics can be estimated before the change by simply answering the questionnaire about the skin concerns.

### <Effects>

In the present invention, the reason (factor) for a change (for example, an improvement) in under-eye sagging can be determined by comparing the volume of the under-eye area of a subject before the change in the under-eye sagging (for example, before the improvement in the under-eye sagging) with the volume of the under-eye area of the subject after the change in the under-eye sagging (for example, after the improvement in the under-eye sagging).

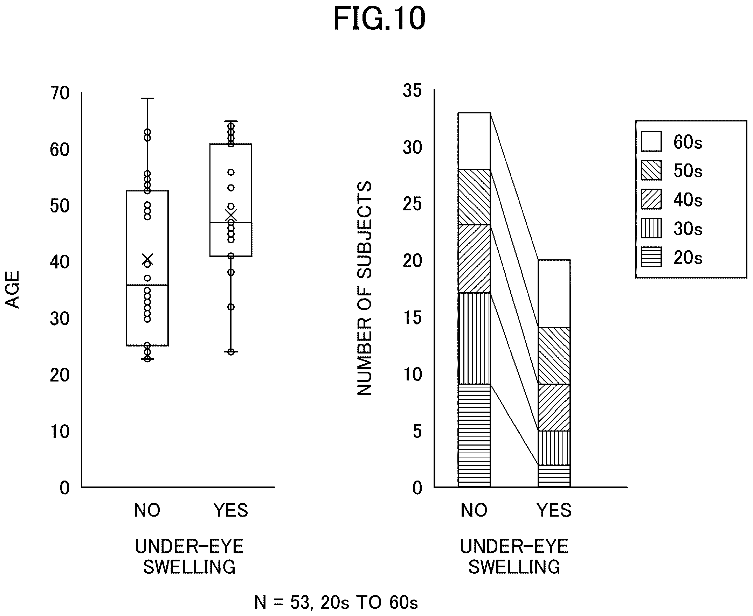

In the following, an example of a method of determining whether under-eye sagging of a subject has changed (for example, improved) will be described. According to an embodiment of the present invention, a method of evaluating under-eye sagging as will be described with reference to FIG. 10 to FIG. 22 can be used to determine whether the under-eye sagging of the subject has changed.

### [Factors that Cause Under-Eye Sagging Appearance in Eyes]

Factors that cause an under-eye sagging appearance will be described with reference to FIG. 10 to FIG. 18.

### <Under-Eye Swelling>

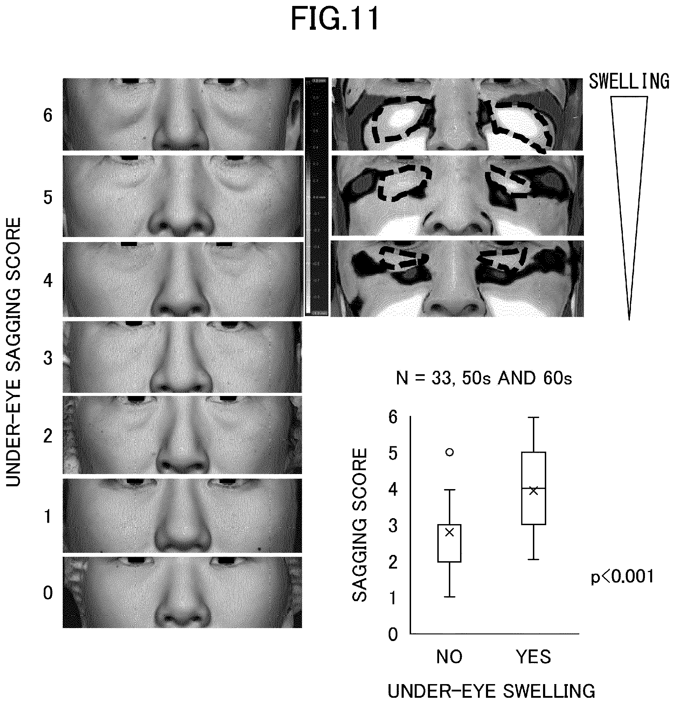

First, "under-eye swelling" will be described with reference to FIG. 10 and FIG.11. The term "under-eye swelling" means that a portion of a subject is more swollen when the subject is in the vertical position than when the subject is in the horizontal position.

FIG. 10 is a drawing illustrating under-eye swelling. Results indicated in FIG. 10 were obtained by examining the presence or absence of under-eye swelling of fifty-three subjects in their 20s to 60s.

A boxplot on the left side of FIG. 10 indicates the ages of subjects who do not have under-eye swelling ("under-eye swelling: NO") and the ages of subjects who have under-eye swelling ("under-eye swelling: YES"). Mainly, the subjects who do not have under-eye swelling ("under-eye swelling: NO") are aged 25 years old to 50 years old, and the subjects who have under-eye swelling ("under-eye swelling: YES") are aged 40 years old to 60 years old.

A bar graph on the right side of FIG. 10 illustrates the number of subjects who do not have under-eye swelling ("under-eye swelling: NO") by age and the number of subjects who have under-eye swelling ("under-eye swelling: YES") by age. The number of subjects in their 50s and 60s who have under-eye swelling is larger than the number of subjects in their 50s and 60s who do not have under-eye swelling. Note that 62% of all the subjects do not have under-eye swelling, and 38% of all the subjects have under-eye swelling.

From the results indicated in FIG. 10, it has been found that some subjects in their 50s and 60s have under-eye swelling and the other subjects in their 50s and 60s do not have under-eye swelling.

FIG. 11 is a drawing illustrating under-eye swelling. Results indicated in FIG. 11 were obtained by examining the presence or absence of under-eye swelling of thirty-three subjects in their 50s and 60s.

A boxplot on the lower right side of FIG. 11 indicates sagging scores of subjects who do not have under-eye swelling ("under-eye swelling: no") and sagging scores of subjects who have under-eye swelling ("under-eye swelling: yes"). Mainly, the subjects who do not have under-eye swelling ("under-eye swelling: no") have sagging scores of 2 and 3, and the subjects who have under-eye swelling ("under-eye swelling: YES") have sagging scores of 3 to 5.

An "under-eye sagging score (also referred to as a visual score (SSeye))" will be described. An "under-eye sagging score (visual score (SSeye)) is a quantified value representing a visual valuation of under-eye sagging (on a seven-grade score from 0 to 6). The under-eye area appears to sag as the value increase.
0: No sagging. Morphology in this area is smooth.
1: Slight sagging. A swollen area is slightly observed near the lower eyelid. Further, a slight hollow area is observed near the inner canthus.
2: Mild sagging. A swollen area and a hollow area are clearly observed.
3: Moderate sagging. A swollen area is enlarged, and a hollow area reaches the midpoint between the inner canthus and the outer canthus.
4: Severe sagging. A swollen area is enlarged, and a hollow area extends beyond the midpoint between the inner canthus and the outer canthus.
5: Very severe sagging. A swollen area extends all over the area, and a hollow area reaches the outer canthus.
6: Extremely severe sagging. A swollen area takes a baggy form.

Based on photographs obtained by capturing the face of a person subject to an evaluation of under-eye sagging at an angle of 45° to the left or to the right, an evaluator determines as to which of standards 0 to 6 the under-eye area of the person subject to the evaluation of under-eye sagging is closest. Then, the evaluator calculates an under-eye sagging score (visual score (SSeye)) based on the determination result. If there are multiple evaluators, a representative value of scores calculated by the evaluators is used. Examples of the representative value include the mean, the mode, and the median. Note that the under-eye sagging score (visual score (SSeye)) of the person subject to the evaluation of under-eye sagging may be calculated by a trained model generated through machine learning in which face images, for which under-eye sagging scores (visual scores (SSeye)) have been determined, are used as training data.

From the results indicated in FIG. 11, it has been found that subjects in their 50s or older and having sagging scores (visual scores (SSeye)) of 4 or more have under-eye swelling and thus their under-eye areas appear to sag.

Next, under-eye sagging scores (visual scores (SSeye)) and age distribution will be described.

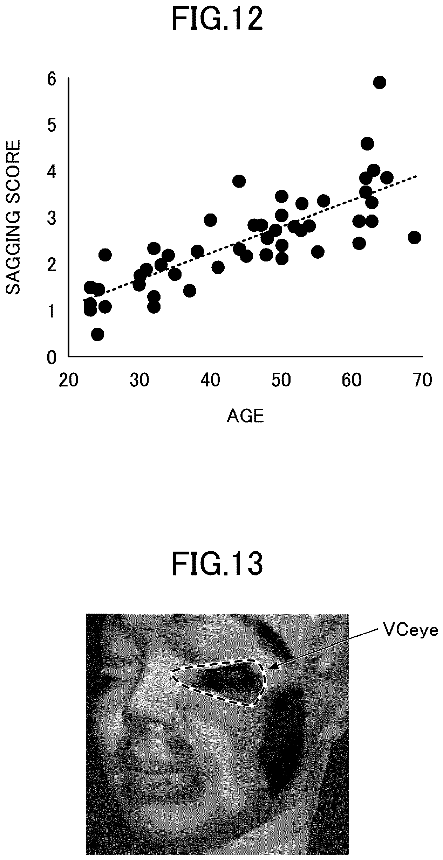

FIG. 12 is a drawing illustrating the relationship between an under-eye sagging score and age. As illustrated in FIG. 12, an under-eye sagging score (visual score (SSeye)) increases (that is, the under-eye area appears to sag more) with age. In FIG. 11, it has been found that the subjects in their 50s or older and having under-eye sagging scores (visual scores (SSeye)) of 4 or more have under-eye swelling and thus their under-eye areas appear to sag.

Results obtained by analyzing the reasons why the under-eye areas of subjects in their 20s to 60s and having under-eye sagging scores (visual scores (SSeye)) of 4 or less appear to sag will be described.

### <Drooping of Upper cheek>

First, "drooping of the upper cheek" will be described with reference to FIG. 13 to FIG. 15. "Drooping of the upper cheek" means that a portion (for example, a portion surrounded by a dotted line in FIG. 13) of a subject is more hollow when the subject is in the vertical position than when the subject is in the horizontal position. The volume (cc) of the portion that is more hollow when the subject is in the vertical position than when the subject is in the horizontal position is denoted as VCeye.

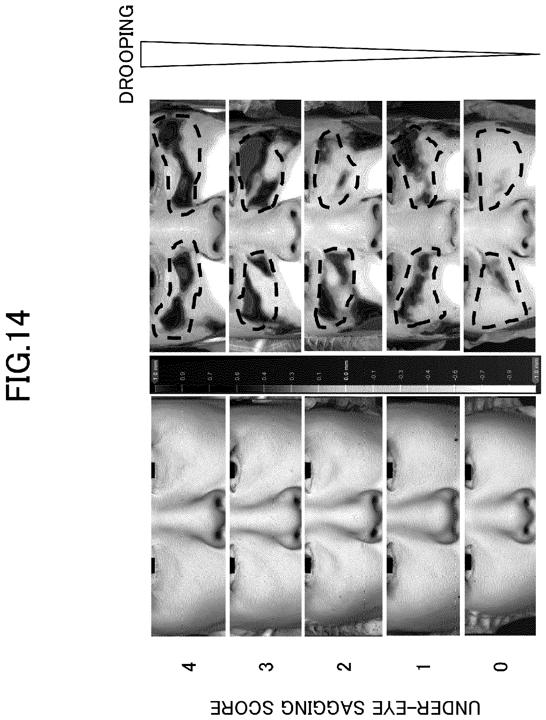

FIG. 14 is a drawing illustrating the relationship between an under-eye sagging score and drooping of the upper cheek. As illustrated in FIG. 14, the greater the VCeye, the higher an under-eye sagging score (visual score (SSeye)).

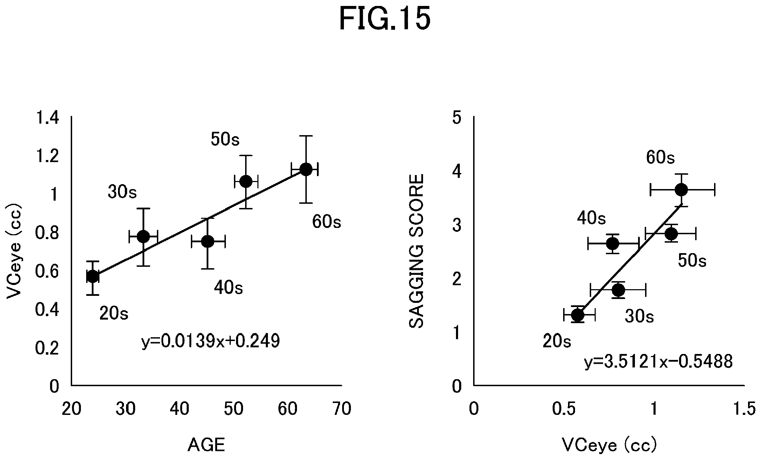

FIG. 15 is a drawing illustrating the relationship between age and the amount (VCeye) of drooping of the upper cheek, and the relationship between the amount (VCeye) of drooping of the upper cheek and an under-eye sagging score. The VCeye and under-eye sagging scores (visual scores (SSeye)) of fifty-three subjects in their 20s to 60s were measured.

The left side of FIG. 15 illustrates the relationship between age and the amount (VCeye) of drooping of the upper cheek. As illustrated in the graph, the amount (VCeye) of drooping of the upper cheek increases with age.

The right side of FIG. 15 illustrates the relationship between the amount (VCeye) of drooping of the upper cheek and an under-eye sagging score. As illustrated in the graph, the greater the amount (VCeye) of drooping of the upper cheek, the higher an under-eye sagging score.

From the results indicated in FIG. 13 to FIG. 15, it has been found that drooping of the upper cheek contributes to an under-eye sagging appearance.

### <Under-Eye Swelling and Drooping of Upper Cheek>

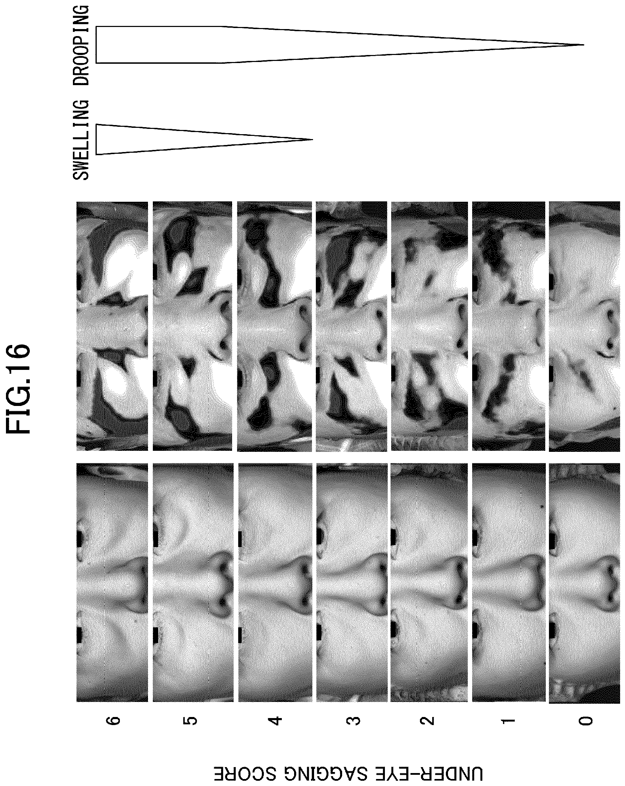

FIG. 16 is a drawing illustrating the relationship between an under-eye sagging score versus under-eye swelling and drooping of the upper cheek. As illustrated in FIG. 16, as an under-eye sagging score (visual score (SSeye)) increases, both under-eye swelling and drooping of the upper cheek become more prominent. Further, as an under-eye sagging score (visual score (SSeye)) increases, the amount of under-eye swelling increases (that is, a difference between the shape of a portion of a subject in the horizontal position and the shape of the portion of the subject in the vertical position increases. The portion of the subject is more swollen when the subject is in the vertical position than when the subject is in the horizontal position.). Further, as an under-eye sagging score (visual score (SSeye)) increases, the amount of drooping of the upper cheek increases (that is, a difference between the shape of a portion of the subject in the horizontal position and the shape of the portion of the subject in the vertical position increases. The portion of the subject is more hollow when the subject is in the vertical position than when the subject is in the horizontal position.).

As described, two factors (that is, "under-eye swelling (protrusion of the eye socket area)" and "drooping of the upper cheek") that cause an under-eye sagging appearance were found. Specifically, it has been found that the under-eye area of a person having the two factors "under-eye swelling" and "drooping of the upper cheek" appears to sag. In addition, it has been found that, for the person having the two factors "under-eye swelling" and "drooping of the upper cheek", the greater the amount of under-eye swelling is, the more the under-eye area appears to sag. In addition, it has been found that, for the person having the two factors "under-eye swelling" and "drooping of the upper cheek", the greater the amount of drooping of the upper cheek is, the more the under-eye area appears to sag.

### <Estimation of Amount (VCeye) of Drooping of Upper Cheek>

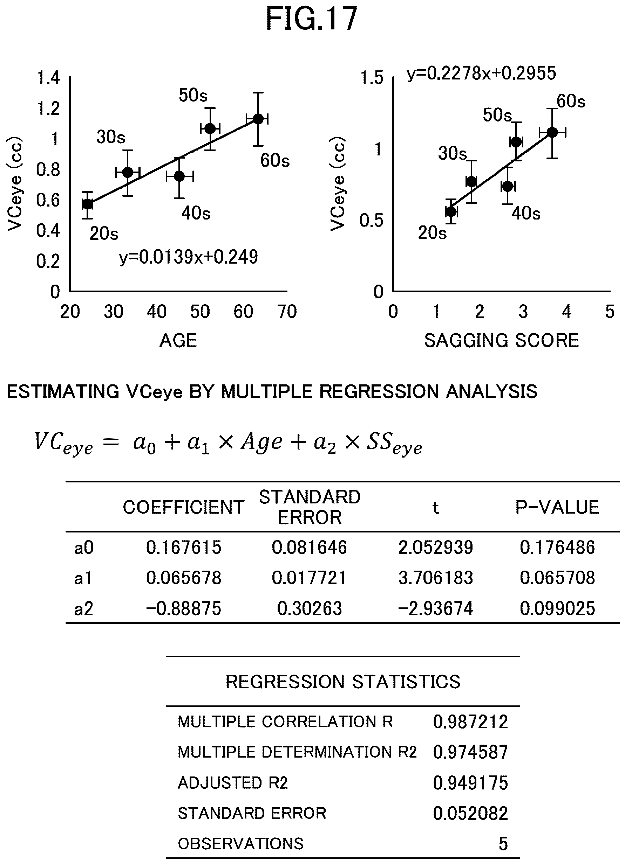

FIG. 17 is a drawing illustrating estimation of the amount (VCeye) of drooping of the upper cheek. The upper left side of FIG. 17 indicates that the amount (VCeye) of drooping of the upper cheek can be estimated from age based on the fact that age is well correlated with the amount (VCeye) of drooping of the upper cheek. A regression equation for estimating the amount (VCeye) of drooping of the upper cheek from age was obtained from a regression line by using coordinates on the upper left side of FIG. 17. The upper right side of FIG. 17 indicates that the amount (VCeye) of drooping of the upper cheek can be estimated from an under-eye sagging score (visual score (SSeye)) based on the fact that the under-eye sagging score (visual score (SSeye)) is well correlated with the amount (VCeye) of drooping of the upper cheek. A regression equation for estimating the amount (VCeye) of drooping of the upper cheek from the under-eye sagging score (visual score (SSeye)) was obtained from a regression line by using coordinates on the upper right side of FIG. 17. The lower part of FIG. 17 indicates that the amount (VCeye) of drooping of the upper cheek can be estimated from age and an under-eye sagging score (visual score (SSeye)) based on the fact that age and the under-eye sagging score (visual score (SSeye)) are well correlated with the amount (VCeye) of drooping of the upper cheek. The lower part of FIG. 17 also indicates a multiple regression equation for estimating the amount (VCeye) of drooping of the upper cheek. Coefficients and regression statistics of the multiple regression equation for estimating the amount (VCeye) of drooping of the upper cheek from age and the under-eye sagging score (visual score (SSeye)) were obtained from multiple regression analysis by using the coordinates on the upper left side of FIG. 17 and the coordinates on the right upper side of FIG. 17.

### <Under-Eye Dark Circle and Pigmentation>

An under-eye sagging score (visual score (SSeye)) is affected by an under-eye skin color including an under-eye dark circle and pigmentation, separately from the two factors (that is, "under-eye swelling (protrusion of the eye socket area)" and "drooping of the upper cheek").

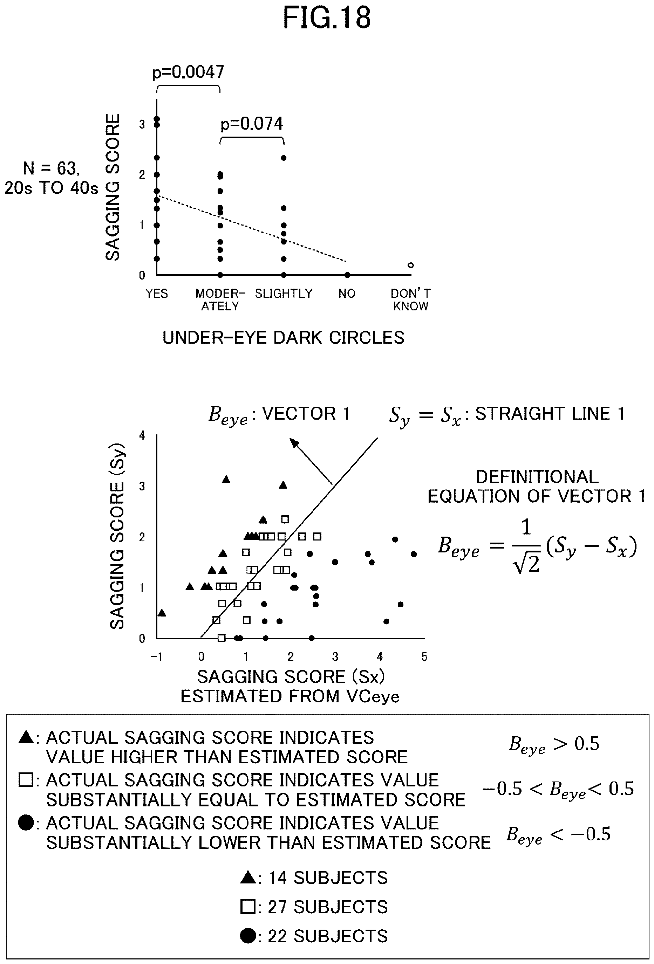

FIG. 18 is a drawing illustrating an influence of an under-eye skin color including an under-eye dark circle and pigmentation on the under-eye sagging score (visual score (SSeye)). A questionnaire was given to sixty-three subjects in their 20s to 40s to ask whether they feel they have dark circles under their eyes, and results indicated in the upper part of FIG. 18 were obtained. Under-eye sagging scores (visual scores (SSeye)) of subjects who answered "yes" were significantly higher than under-eye sagging scores (visual scores (SSeye)) of subjects who answered "moderately". This indicates that an under-eye sagging score (visual score (SSeye)) is evaluated to be high due to an under-eye skin color including an under-eye dark circle and pigmentation.

The lower part of FIG. 18 illustrates results obtained by comparing an estimated under-eye sagging score (visual score (SSeye)) estimated from VCeye with an actual under-eye sagging score (visual score (SSeye)), based on the fact that an under-eye sagging score (visual score (SSeye)) is evaluated to be high due to an under-eye skin color including an under-eye dark circle and pigmentation. In the lower part of FIG. 18, based on a vector 1 (definitional equation 1) perpendicular to a straight line 1, there are three groups: a group (black triangle) in which an actual sagging score indicates a value higher than an estimated under-eye sagging score (visual score (SSeye)) estimated from VCeye; a group (white square) in which an actual sagging score indicates a value substantially equal to an estimated under-eye sagging score (visual score (SSeye)) estimated from VCeye ; and a group (black circle) in which an actual sagging score indicates a value lower than an estimated under-eye sagging score (visual score (SSeye)) estimated from VCeye.

In the group (black triangle) in which an actual sagging score indicates a value higher than an estimated under-eye sagging score (visual score (SSeye)) estimated from VCeye, a sagging score is evaluated to be high due to an under-eye skin color including an under-eye dark circle and pigmentation. Therefore, a treatment for not only improving the two factors, which cause an under-eye sagging appearance, but also improving an under-eye skin color including an under-eye dark circle and pigmentation can be recommended.

In the group (black circle) in which an actual sagging score indicates a value smaller than an estimated under-eye sagging score (visual score (SSeye)) estimated from VCeye, an under-eye sagging appearance is not noticeable, but "drooping of the upper cheek", which causes a sagging appearance, is prominent. Thus, a treatment for improving "drooping of the upper cheek" before the symptom worsens and becomes apparent can be recommended.

### [Evaluation of Under-Eye Sagging]

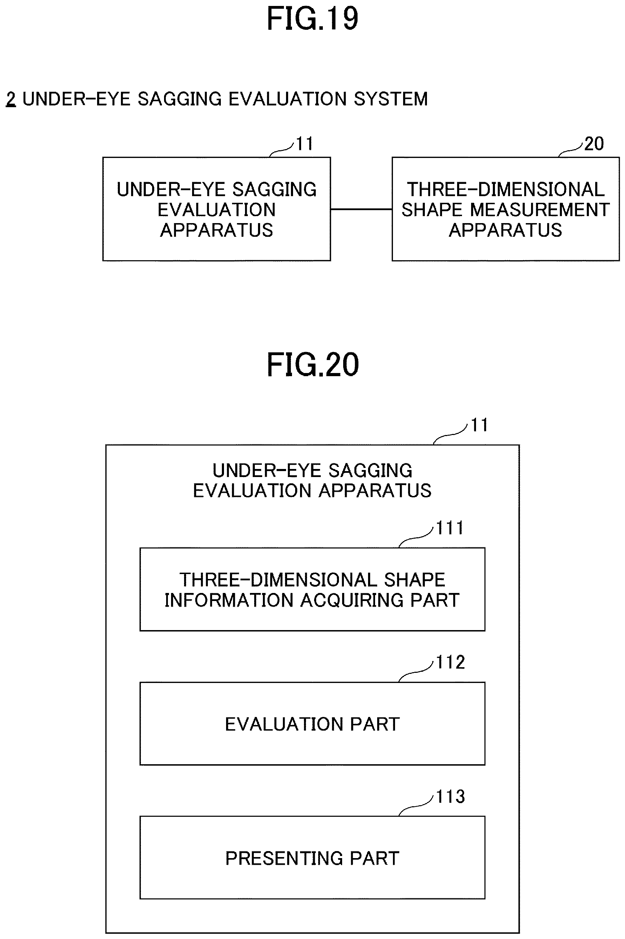

An evaluation of under-eye sagging of a subject (a person subject to an evaluation of under-eye sagging) will be described with reference to FIG. 19 to FIG. 22. Specifically, an evaluation of under-eye sagging that uses the two factors "under-eye swelling", which means that the under-eye area of the subject is more swollen when the subject is in the vertical position than when the subject is in the horizontal position, and "drooping of the upper cheek", which means that the upper cheek is more hollow when the subject is in the vertical position than when the subject is in the horizontal position, will be described.

### <Overall Configuration>

FIG. 19 is a block diagram of an under-eye sagging evaluation system 2 according to an embodiment of the present invention.
The under-eye sagging evaluation system 2 includes an under-eye sagging evaluation apparatus 11 and a three-dimensional shape measurement apparatus 20.

The under-eye sagging evaluation apparatus 11 is a computer (for example, a personal computer, a tablet, a smartphone, a server, or the like) configured to determine the reason for a change in under-eye sagging. Specifically, the under-eye sagging evaluation apparatus 11 is configured to evaluate at least one of under-eye swelling and drooping of the upper cheek based on a difference (that is, a change) between the three-dimensional shape of the under-eye area of a subject when the subject is in the horizontal position and the three-dimensional shape of the under-eye area of the subject when the subject is in the vertical position, and evaluate under-eye sagging based on at least one of the under-eye swelling and the drooping of the upper cheek. The under-eye sagging evaluation apparatus 11 may be implemented by the same computer as the reason-for-change in under-eye sagging determination apparatus 10. The under-eye sagging evaluation apparatus 11 will be described later in detail with reference to FIG. 20.

Although the under-eye sagging evaluation apparatus 11 is described as one computer in FIG. 19, the under-eye sagging evaluation apparatus 11 may be implemented by a plurality of computers (for example, a terminal such as a smartphone used by the subject, and a server that can transmit/receive data to/from the terminal. For example, the terminal such as the smartphone used by the subject may transmit, to the server, information on the three-dimensional shapes of the under-eye area of the subject when the subject is in the horizontal position and the vertical position, and may receive, from the server, an evaluation result of under-eye sagging evaluated by the server.

The three-dimensional shape measurement apparatus 20 is an apparatus configured to measure the three-dimensional shape of the under-eye area of the subject. The three-dimensional shape measurement apparatus 20 can generate an image representing the three-dimensional shape of the face including at least the under-eye area of the subject. Specifically, the three-dimensional shape measurement apparatus 20 generates an image representing the three-dimensional shape of the face including at least the under-eye area of the subject when the subject is in the horizontal position (specifically, when the median plane of the face rests at right angles to the direction of gravity), and an image representing the three-dimensional shape of the face including at least the under-eye area of the subject when the subject is in the vertical position (specifically, when the median plane of the face rests parallel to the direction of gravity).

### <Functional Block>

FIG. 20 is a functional block diagram of the under-eye sagging evaluation apparatus 11 according to an embodiment of the present invention. The under-eye sagging evaluation apparatus 11 can include a three-dimensional shape information acquiring part 111, an evaluation part 112, and a presenting part 113. Further, the under-eye sagging evaluation apparatus 11 can function as the three-dimensional shape information acquiring part 111, the evaluation part 112, and the presenting part 113 by executing a program. Each of the parts will be described below.

The three-dimensional shape information acquiring part 111 acquires information on the three-dimensional shape of the under-eye area of the subject when the subject is in the horizontal position and information on the three-dimensional shape of the under-eye area of the subject when the subject is in the vertical position. For example, the three-dimensional shape information acquiring part 111 acquires, from the three-dimensional shape measurement apparatus 20, an image representing the three-dimensional shape of the face including at least the under-eye area of the subject when the subject is in the horizontal position, and an image representing the three-dimensional shape of the face including at least the under-eye area of the subject when the subject is in the vertical position. The three-dimensional shape information acquiring part 111 stores the acquired information on the three-dimensional shapes of the under-eye area of the subject in a memory so that the evaluation part 112 can refer to the information.

The evaluation part 112 evaluates under-eye sagging of the subject based on the information on the three-dimensional shapes of the under-eye area of the subject acquired by the three-dimensional shape information acquiring part 111. In the following, an evaluation of under-eye swelling and drooping of the upper cheek, and an evaluation of under-eye sagging based on the under-eye swelling and the drooping of the upper cheek will be described separately.

### <<Evaluation of Under-Eye Swelling and Drooping of Upper Cheek>>

The evaluation part 112 evaluates at least one of under-eye swelling and drooping of the upper cheek based on a difference between the three-dimensional shape of the under-eye area of the subject when the subject is in the horizontal position and the three-dimensional shape of the under-eye area of the subject when the subject is in the vertical position.

Specifically, the evaluation part 112 calculates, as the amount of under-eye swelling, a difference between the shape of a portion of the subject when the subject is in the horizontal position and the shape of the portion of the subject when the subject is in the vertical position. The portion of the subject is more swollen when the subject is in the vertical position than when the subject is in the horizontal position. Further, the evaluation part 112 calculates, as the amount of drooping of the upper cheek, a difference between the shape of a portion of the subject when the subject is in the horizontal position and the shape of the portion of the subject when the subject is in the vertical position. The portion of the subject is more hollow when the subject is in the vertical position than when the subject is in the horizontal position.

### <<Evaluation of Under-Eye Sagging>>

The evaluation part 112 evaluates under-eye sagging based on at least one of the under-eye swelling and the drooping of the upper cheek.

Specifically, in a case where both the under-eye swelling and the drooping of the upper cheek are present in the under-eye area, the evaluation part 112 can evaluate that the under-eye area is sagging. Further, in a case where both the under-eye swelling and the drooping of the upper cheek are present in the under-eye area, the evaluation part 112 can evaluate that the under-eye area is sagging as the amount of the under-eye swelling increases. Further, in a case where both the under-eye swelling and the drooping of the upper cheek are present in the under-eye area, the evaluation part 112 can evaluate that the under-eye area is sagging as the amount of the drooping of the upper cheek increases.

The presenting part 113 presents cosmetics or a beauty treatment in accordance with the evaluation of the under-eye sagging performed by the evaluation part 112 (for example, displays cosmetics or a beauty treatment on a display unit of the under-eye sagging evaluation apparatus 11 or another apparatus). Further, the presenting part 113 can present results of the evaluation performed by the evaluation part 112 (for example, display results of the evaluation on a display unit of the under-eye sagging evaluation apparatus 11 or another apparatus). Note that the presenting part 103 can present an image in which results of the evaluation of the under-eye sagging are superimposed on a photograph of the face of the subject.

### <Method>

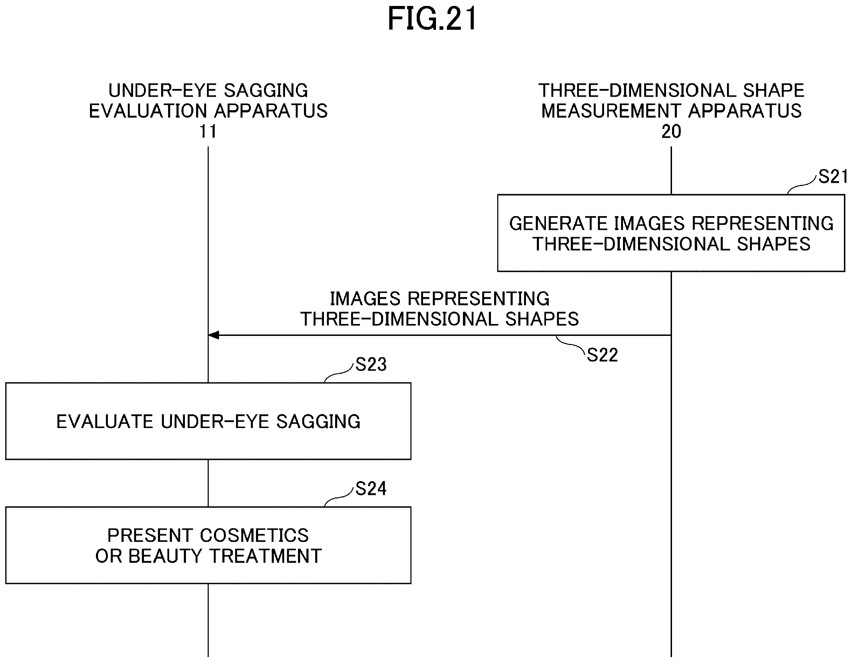

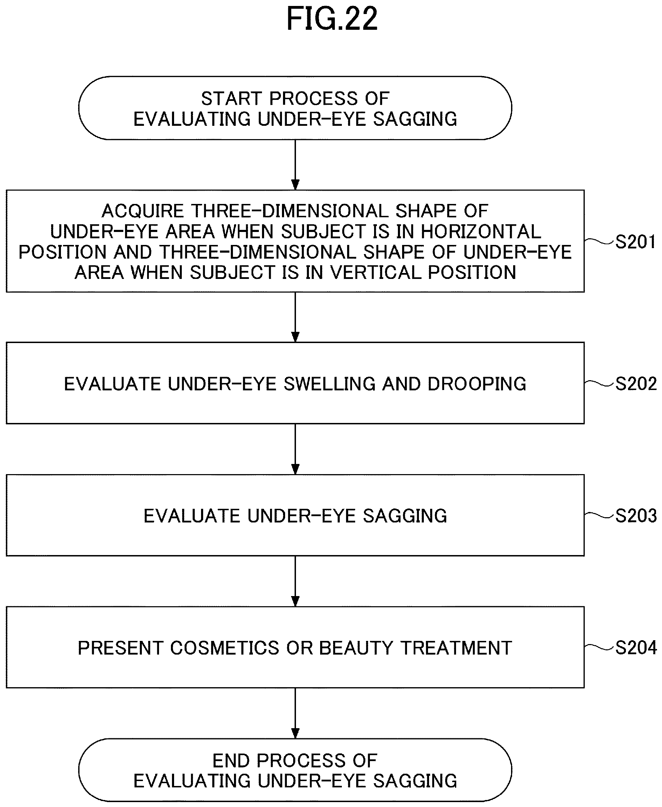

A method of evaluating under-eye sagging will be described below with reference to FIG. 21 and FIG. 22.

FIG. 21 is a sequence diagram of a process of evaluating under-eye sagging according to an embodiment of the present invention.

In step 21 (S21), the three-dimensional shape measurement apparatus 20 generates an image representing the three-dimensional shape of the face including at least the under-eye area of the subject when the subject is in the horizontal position (specifically, when the median plane of the face rests at right angles to the direction of gravity), and an image representing the three-dimensional shape of the face including at least the under-eye area of when the subject is in the vertical position (specifically, when the median plane of the face rests parallel to the direction of gravity).

In step 22 (S22), the under-eye sagging evaluation apparatus 11 acquires, from the three-dimensional shape measurement apparatus 20, the images (images generated in S21) representing the three-dimensional shapes of the face including at least the under-eye area of the subject when the subject is in the horizontal position and when the subject is in the vertical position.

In step 23 (S23), the under-eye sagging evaluation apparatus 11 evaluates under-eye sagging of the subject based on information on the three-dimensional shapes of the under-eye area of the subject acquired in S22.

In step 24 (S24), the under-eye sagging evaluation apparatus 11 presents cosmetics or a beauty treatment in accordance with the evaluation of the under-eye sagging in S23 (for example, displays cosmetics or a beauty treatment on a display unit of the under-eye sagging evaluation apparatus 11 or another apparatus).

FIG. 22 is a flowchart of the process of evaluating under-eye sagging according to an embodiment of the present invention.

In step 201 (S201), the three-dimensional shape information acquiring part 111 of the under-eye sagging evaluation apparatus 11 acquires information on the three-dimensional shape of the under-eye area of the subject when the subject is in the horizontal position and information on the three-dimensional shape of the under-eye area of the subject when the subject is in the vertical position. For example, the three-dimensional shape information acquiring part 111 acquires, from the three-dimensional shape measurement apparatus 20, an image representing the three-dimensional shape of the face including at least the under-eye area of the subject when the subject is in the horizontal position, and an image representing the three-dimensional shape of the face including at least the under-eye area of the subject when the subject is in the vertical position.

In step 202 (S202), the evaluation part 112 of the under-eye sagging evaluation apparatus 11 evaluates at least one of under-eye swelling and drooping of the upper cheek based on a difference between the three-dimensional shape of the under-eye area of the subject when the subject is in the horizontal position and the three-dimensional shape of the under-eye area of the subject when the subject is in the vertical position, which are acquired in S201.

Specifically, the evaluation part 112 calculates, as the amount of under-eye swelling, a difference between the shape of a portion of the subject when the subject is in the horizontal position and the shape of the portion of the subject when the subject is in the vertical position, which are acquired in S201. The portion of the subject is more swollen when the subject is in the vertical position than when the subject is in the horizontal position. Further, the evaluation part 112 calculates, as the amount of drooping of the upper cheek, a difference between the shape of a portion of the subject when the subject is in the horizontal position and the shape of the portion of the subject when the subject is in the vertical position, which are acquired in S201. The portion of the subject is more hollow when the subject is in the vertical position than when the subject is in the horizontal position.

In step 203 (S203), the evaluation part 112 of the under-eye sagging evaluation apparatus 11 evaluates under-eye sagging based on at least one of the under-eye swelling and the drooping of the upper cheek, which are evaluated in S202.

Specifically, in a case where both the under-eye swelling and the drooping of the upper cheek are present in the under-eye area, the evaluation part 112 can evaluate that the under-eye area is sagging. Further, in a case where both the under-eye swelling and the drooping of the upper cheek are present in the under-eye area, the evaluation part 112 can evaluate that the under-eye area is sagging as the amount of the under-eye swelling increases. Further, in a case where both the under-eye swelling and the drooping of the upper cheek are present in the under-eye area, the evaluation part 112 can evaluate that the under-eye area is sagging as the amount of the drooping of the upper cheek increases.

In step 204 (S204), the presenting part 113 of the under-eye sagging evaluation apparatus 11 presents cosmetics or a beauty treatment in accordance with the evaluation of the under-eye sagging in S203 (for example, displays cosmetics or a beauty treatment on a display unit of the under-eye sagging evaluation apparatus 11).

### <Effects>

In the present invention, under-eye sagging can be evaluated by using the two factors "under-eye swelling", which means that the under-eye area is more swollen when the subject is in the horizontal position than when the subject is in the vertical position, and "drooping of the upper cheek", which means that the upper cheek is more hollow when the subject is in the horizontal position than when the subject is in the vertical position. Accordingly, a more objective evaluation can be performed than a visual valuation.

### <Hardware Configuration>

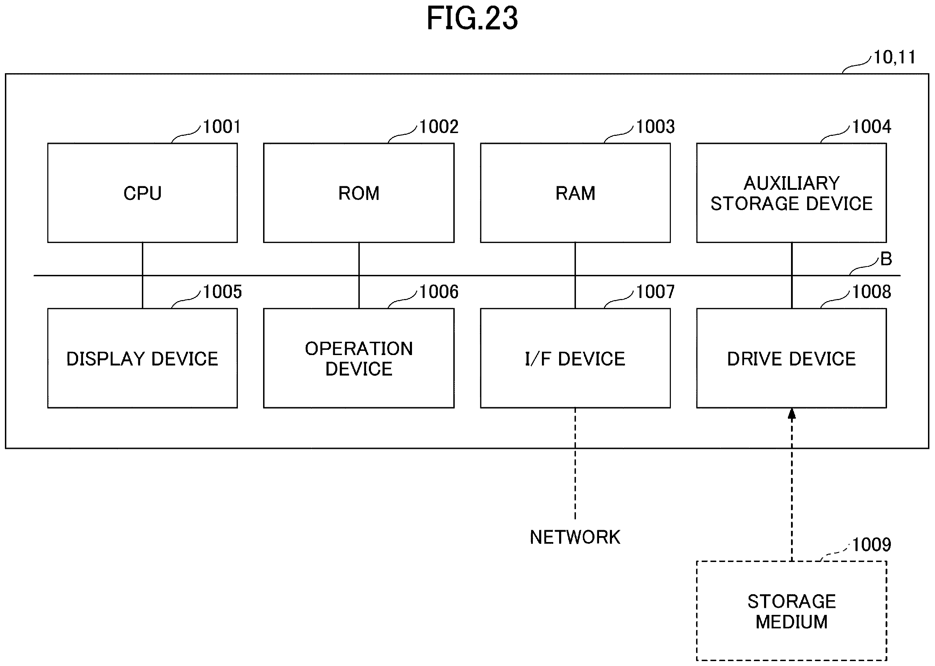

FIG. 23 is a block diagram illustrating an example of a hardware configuration of each of the reason-for-change in under-eye sagging determination apparatus 10 and the under-eye sagging evaluation apparatus 11 according to an embodiment of the present disclosure. Each of the reason-for-change in under-eye sagging determination apparatus 10 and the under-eye sagging evaluation apparatus 11 includes a central processing unit (CPU) 1001, a read-only memory(ROM) 1002, and a random access memory (RAM) 1003. The CPU 1001, the ROM 1002, and the RAM 1003 form what is known as a computer.

Further, each of the reason-for-change in under-eye sagging determination apparatus 10 and the under-eye sagging evaluation apparatus 11 can include an auxiliary storage device 1004, a display device 1005, an operation device 1006, an interface (I/F) device 1007, and a drive device 1008. The hardware components of each of the reason-for-change in under-eye sagging determination apparatus 10 and the under-eye sagging evaluation apparatus 11 are connected to one another via a bus B.

The CPU 1001 is an arithmetic device that executes various programs installed in the auxiliary storage device 1004.

The ROM 1002 is a non-volatile memory. The ROM 1002 functions as a main storage device that stores various programs, data, and the like necessary for the CPU 1001 to execute the various programs installed in the auxiliary storage device 1004. Specifically, the ROM 1002 functions as a main storage device that stores boot programs such as a basic input/output system (BIOS) and an extensible firmware interface (EFI).

The RAM 1003 is a volatile memory such as a dynamic random access memory (DRAM) and a static random access memory (SRAM). The RAM 1003 functions as a main storage device that provides a working area developed when the various programs installed in the auxiliary storage device 1004 are executed by the CPU 1001.

The auxiliary storage device 1004 is an auxiliary storage device that stores various programs and information used when the various programs are executed.

The display device 1005 is a display device that displays, for example, an internal state, and the like of each of the reason-for-change in under-eye sagging determination apparatus 10 and the under-eye sagging evaluation apparatus 11.

The operation device 1006 is an input device with which an administrator of the reason-for-change in under-eye sagging determination apparatus 10 and the under-eye sagging evaluation apparatus 11 inputs various instructions into the reason-for-change in under-eye sagging determination apparatus 10 and the under-eye sagging evaluation apparatus 11.

The I/F device 1007 is a communication device that is connected to a network and is for communicating with other devices.

The drive device 1008 is a device for setting the storage medium 1009. The storage medium 1009 herein includes a medium for optically, electrically, or magnetically recording information, such as a CD-ROM, a flexible disk, or a magneto-optical disc. Further, the storage medium 1009 may include a semiconductor memory or the like that electrically records information, such as an erasable programmable read-only memory (EPROM), a flash memory, or the like.

Note that the various programs installed in the auxiliary storage device 1004 are installed when, for example, the distributed storage medium 1009 is set in the drive device 1008 and various programs recorded in the storage medium 1009 are read out by the drive device 1008. Alternatively, the various programs installed in the auxiliary storage device 1004 may be installed by being downloaded from the network via the I/F device 1007.

Although embodiments of the present invention have been described in detail above, the present invention is not limited to the specific embodiments described above, and various modifications and changes can be made without departing from the scope of the claims.

This international application claims priority based on Japanese Patent Application No. 2021-107017, filed on June 28, 2021, and Japanese Patent Application No. 2021-107018, filed on June 28, 2021. The entire contents of Japanese Patent Application No. 2021-107017 and Japanese Patent Application No. 2021-107018 are incorporated herein by reference.

### DESCRIPTION OF THE REFERENCE NUMERALS

1 reason-for-change in under-eye sagging determination system
10 reason-for-change in under-eye sagging determination apparatus
20 three-dimensional shape measurement apparatus
101 three-dimensional shape information acquiring part
102 determination part
103 presenting part
2 under-eye sagging evaluation system
11 under-eye sagging evaluation apparatus
111 three-dimensional shape information acquiring part
112 evaluation part
113 presenting part
1001 CPU
1002 ROM
1003 RAM
1004 auxiliary storage device
1005 display device
1006 operation device
1007 I/F device
1008 drive device
1009 storage medium

## Claims

1. A reason-for-change in under-eye sagging determination method comprising:
acquiring information on a three-dimensional shape of an under-eye area of a subject when the subject is in a vertical position before a change in under-eye sagging of the subject and information on a three-dimensional shape of the under-eye area of the subject when the subject is in the vertical position after the change in the under-eye sagging; and
determining a reason for the change in the under-eye sagging, based on a volume difference between the three-dimensional shape of the under-eye area of the subject when the subject is in the vertical position before the change in the under-eye sagging and the three-dimensional shape of the under-eye area of the subject when the subject is in the vertical position after the change in the under-eye sagging.

2. The reason-for-change in under-eye sagging determination method according to claim 1, wherein, in a case where a volume of the under-eye area is smaller after the change in the under-eye sagging than before the change in the under-eye sagging, it is determined that the under-eye sagging has changed as a result of disappearance of edema originally observed in the subject and causing the under-eye sagging.

3. The reason-for-change in under-eye sagging determination method according to claim 1, wherein, in a case where a volume of the under-eye area is larger after the change in the under-eye sagging than before the change in the under-eye sagging, it is determined that the under-eye sagging has changed as a result of an improvement in loss of elasticity originally observed in the subject and causing the under-eye sagging.

4. The reason-for-change in under-eye sagging determination method according to claim 1, wherein, in a case where a volume of the under-eye area is not determined to be larger or smaller after the change in the under-eye sagging than before the change in the under-eye sagging, it is determined that the under-eye sagging has changed as a result of a change in an under-eye skin color, including at least one of an under-eye dark circle and pigmentation, originally observed in the subject and causing the under-eye sagging.

5. The reason-for-change in under-eye sagging determination method according to any one of claims 1 to 4, further comprising
determining whether the under-eye sagging of the subject has changed by
acquiring information on a three-dimensional shape of an under-eye area including an upper cheek of the subject when the subject is in the horizontal position and information on a three-dimensional shape of the under-eye area including the upper cheek of the subject when the subject is in the vertical position; and
evaluating at least one of under-eye swelling and drooping of the upper cheek, based on a difference between the three-dimensional shape of the under-eye area including the upper cheek of the subject when the subject is in the horizontal position and the three-dimensional shape of the under-eye area including the upper cheek of the subject when the subject is in the vertical position, and
evaluating the under-eye sagging based on at least one of the under-eye swelling and the drooping of the upper cheek.

6. The reason-for-change in under-eye sagging determination method according to claim 5, wherein, in a case where both the under-eye swelling and the drooping of the upper cheek are present in the under-eye area, it is evaluated that the under-eye area is sagging.

7. The reason-for-change in under-eye sagging determination method according to claim 5 or 6, wherein an amount of the under-eye swelling is a difference between a shape of a portion of the subject when the subject is in the horizontal position and a shape of the portion of the subject when the subject is in the vertical position, the portion being more swollen when the subject is in the vertical position than when the subject is in the horizontal position, and
it is evaluated that the under-eye area is sagging as the amount of the under-eye swelling increases.

8. The reason-for-change in under-eye sagging determination method according to any one of claims 5 to 7, wherein an amount of the drooping of the upper cheek is a difference between a shape of a portion of the subject when the subject is in the horizontal position and a shape of the portion of the subject when the subject is in the vertical position, the portion being more hollow when the subject is in the vertical position than when the subject is in the horizontal position, and
it is evaluated that the under-eye area is sagging as the amount of the drooping of the upper cheek increases.

9. The reason-for-change in under-eye sagging determination method according to claim 5, wherein an amount (VCeye) of the drooping of the upper cheek estimated from age based on a fact that the age is correlated with the amount (VCeye) of the drooping of the upper cheek is used.

10. The reason-for-change in under-eye sagging determination method according to claim 5, wherein an amount (VCeye) of the drooping of the upper cheek estimated from an under-eye sagging score based on a fact that the under-eye sagging score is correlated with the amount (VCeye) of the drooping of the upper cheek is used.

11. The reason-for-change in under-eye sagging determination method according to claim 5, wherein an amount (VCeye) of the drooping of the upper cheek estimated from both age and an under-eye sagging score based on a fact that the age is correlated with the amount (VCeye) of the drooping of the upper cheek and that the under-eye sagging score is correlated with the amount (VCeye) of the drooping of the upper cheek is used.

12. The reason-for-change in under-eye sagging determination method according to claim 5, further comprising
evaluating that the under-eye area appears to be sagging due to a change in an under-eye skin color including at least one of an under-eye dark circle and pigmentation, in a case where an actual under-eye sagging score (visual score (SSeye)) is higher than an estimated under-eye sagging score (visual score (SSeye)) estimated from VCeye.

13. The reason-for-change in under-eye sagging determination method according to claim 5, further comprising
evaluating that the drooping of the upper cheek is more prominent than an under-eye sagging appearance, in a case where an actual under-eye sagging score (visual score (SSeye)) is lower than an estimated under-eye sagging score (visual score (SSeye)) estimated from VCeye.

14. The reason-for-change in under-eye sagging determination method according to claim 1, further comprising:
generating a correlation between the determined reason for the change in the under-eye sagging and an answer to a predetermined questionnaire, the answer being given by the subject; and
using the correlation to estimate a reason for a change in under-eye sagging based on an answer to the questionnaire.

15. The reason-for-change in under-eye sagging determination method according to any one of claims 1 to 14, wherein the change in the under-eye sagging is an improvement in the under-eye sagging.

16. A reason-for-change in under-eye sagging determination apparatus comprising:
a three-dimensional shape information acquiring part configured to acquire information on a three-dimensional shape of an under-eye area of a subject when the subject is in a vertical position before a change in under-eye sagging of the subject and information on a three-dimensional shape of the under-eye area of the subject when the subject is in the vertical position after the change in the under-eye sagging; and
a determination part configured to determine a reason for the change in the under-eye sagging, based on a volume difference between the three-dimensional shape of the under-eye area of the subject when the subject is in the vertical position before the change in the under-eye sagging and the three-dimensional shape of the under-eye area of the subject when the subject is in the vertical position after the change in the under-eye sagging.

17. A program for causing a reason-for-change in under-eye sagging determination apparatus to function as:
a three-dimensional shape information acquiring part configured to acquire information on a three-dimensional shape of an under-eye area of a subject when the subject is in a vertical position before a change in under-eye sagging of the subject and information on a three-dimensional shape of the under-eye area of the subject when the subject is in the vertical position after the change in the under-eye sagging; and
a determination part configured to determine a reason for the change in the under-eye sagging, based on a volume difference between the three-dimensional shape of the under-eye area of the subject when the subject is in the vertical position before the change in the under-eye sagging and the three-dimensional shape of the under-eye area of the subject when the subject is in the vertical position after the change in the under-eye sagging.

18. An under-eye sagging evaluation method comprising:
acquiring information on a three-dimensional shape of an under-eye area including an upper cheek of a subject when the subject is in a horizontal position and information on a three-dimensional shape of the under-eye area including the upper cheek of the subject when the subject is in a vertical position;
evaluating at least one of under-eye swelling and drooping of the upper cheek based on a difference between the three-dimensional shape of the under-eye area including the upper cheek of the subject when the subject is in the horizontal position and the three-dimensional shape of the under-eye area including the upper cheek of the subject when the subject is in the vertical position; and
performing an evaluation of under-eye sagging based on at least one of the under-eye swelling and the drooping of the upper cheek.

19. The under-eye sagging evaluation method according to claim 18, wherein, in a case where both the under-eye swelling and the drooping of the upper cheek are present in the under-eye area, it is evaluated that the under-eye area is sagging.

20. The under-eye sagging evaluation method according to claim 18 or 19, wherein an amount of the under-eye swelling is a difference between a shape of a portion of the subject when the subject is in the horizontal position and a shape of the portion of the subject when the subject is in the vertical position, the portion being more swollen when the subject is in the vertical position than when the subject is in the horizontal position, and
it is evaluated that the under-eye area is sagging as the amount of the under-eye swelling increases.

21. The under-eye sagging evaluation method according to any one of claims 18 to 20, wherein an amount of the drooping of the upper cheek is a difference between a shape of a portion of the subject when the subject is in the horizontal position and a shape of the portion of the subject when the subject is in the vertical position, the portion being more hollow when the subject is in the vertical position than when the subject is in the horizontal position, and
it is evaluated that the under-eye area is sagging as the amount of the drooping of the upper cheek increases.

22. The under-eye sagging evaluation method according to claim 18, wherein an amount (VCeye) of the drooping of the upper cheek estimated from age based on a fact that the age is correlated with the amount (VCeye) of the drooping of the upper cheek is used.

23. The under-eye sagging evaluation method according to claim 18, wherein an amount (VCeye) of the drooping of the upper cheek estimated from an under-eye sagging score based on a fact that the under-eye sagging score is correlated with the amount (VCeye) of the drooping of the upper cheek is used.

24. The under-eye sagging evaluation method according to claim 18, wherein an amount (VCeye) of the drooping of the upper cheek estimated from both age and an under-eye sagging score based on a fact that the age is correlated with the amount (VCeye) of the drooping of the upper cheek and that the under-eye sagging score is correlated with the amount (VCeye) of the drooping of the upper cheek is used.

25. The under-eye sagging evaluation method according to claim 18, further comprising
evaluating that the under-eye area appears to be sagging due to a change in an under-eye skin color including at least one of an under-eye dark circle and pigmentation, in a case where an actual under-eye sagging score (visual score (SSeye)) is higher than an estimated under-eye sagging score (visual score (SSeye)) estimated from VCeye.

26. The under-eye sagging evaluation method according to claim 18, further comprising
evaluating that the drooping of the upper cheek is more prominent than an under-eye sagging appearance, in a case where an actual under-eye sagging score (visual score (SSeye)) is lower than an estimated under-eye sagging score (visual score (SSeye)) estimated from VCeye.

27. The under-eye sagging evaluation method according to any one of claims 18 to 26, further comprising
presenting a cosmetic or a beauty treatment in according with the evaluation of the under-eye sagging.

28. The under-eye sagging evaluation method according to any one of claims 18 to 27, further comprising
presenting an image in which a result of the evaluation of the under-eye sagging is superimposed on a photograph of a face of the subject.

29. An under-eye sagging evaluation apparatus comprising:
a three-dimensional shape information acquiring part configured to acquire information on a three-dimensional shape of an under-eye area including an upper cheek of a subject when the subject is in a horizontal position and information on a three-dimensional shape of the under-eye area including the upper cheek of the subject when the subject is in a vertical position; and
an evaluation part configured to
evaluate at least one of under-eye swelling and drooping of the upper cheek, based on a difference between the three-dimensional shape of the under-eye area including the upper cheek of the subject when the subject is in the horizontal position and the three-dimensional shape of the under-eye area including the upper cheek of the subject when the subject is in the vertical position, and
evaluate under-eye sagging based on at least one of the under-eye swelling and the drooping of the upper cheek.

30. A program for causing an under-eye sagging evaluation apparatus to function as:
a three-dimensional shape information acquiring part configured to acquire information on a three-dimensional shape of an under-eye area including an upper cheek of a subject when the subject is in a horizontal position and information on a three-dimensional shape of the under-eye area including the upper cheek of the subject when the subject is in a vertical position; and
an evaluation part configured to
evaluate at least one of under-eye swelling and drooping of the upper cheek, based on a difference between the three-dimensional shape of the under-eye area including the upper cheek of the subject when the subject is in the horizontal position and the three-dimensional shape of the under-eye area including the upper cheek of the subject when the subject is in the vertical position, and
evaluate under-eye sagging based on at least one of the under-eye swelling and the drooping of the upper cheek.
